# EUROPEAN PATENT APPLICATION

(11) **EP 3 909 970 A1**
(43) Date of publication of application: **17.11.2021**
(21) Application number: 20382406.5
(22) Date of filing: 14.05.2020
(51) Int. Cl.: C07K 14/09, A61K 39/12, A61K 47/64, A61P 31/14

(54) **PEPTIDE VACCINES FOR THE PREVENTION OF FOOT-AND-MOUTH DISEASE**

(71) Applicant: Consejo Superior De Investigaciones Científicas, 28006 Madrid (ES); Instituto Nacional de Investigación y Tecnologia Agraria y Alimentaria, 28040 Madrid (ES); Universitat Pompeu Fabra, 08002 Barcelona (ES)
(72) Inventor: CAÑAS ARRANZ, Rodrigo, E-28006 Madrid (ES); DE LEÓN VALDÉS, Patricia, E-28006 Madrid (ES); TORRES LORITE, Elisa, E-28006 Madrid (ES); BUSTOS SÁNCHEZ, María José, E-28006 Madrid (ES); SOBRINO CASTELLÓ, Francisco, E-28006 Madrid (ES); BLANCO LAVILLA, Esther, E-28040 Madrid (ES); ANDREU MARTÍNEZ, David, E-08002 Barcelona (ES); DEFAUS FORNAGUER, Sira, E-08002 Barcelona (ES); FORNER CASTELLSAGUÉ, Mar, E-08002 Barcelona (ES)
(74) Representative: ABG Intellectual Property Law, S.L.

(57) **Abstract**

The invention relates to a dendrimeric peptide construct of formula (I) wherein: T is a T epitope of the non-structural protein 3D of the FMDV, bound to the lysine core by its N-terminal end, B is a B epitope of the FMDV, W is a linker group, and an acyl group when B is bound to the α-amino group of the Cys by its C-terminal end, or is either -OH or is a -NH₂ group when B is bound to the α-carboxylic group of the Cys by its N-terminal end. The invention also refers to the use of said constructs as an immunogen for the prevention of foot-and-mouth disease in an animal.

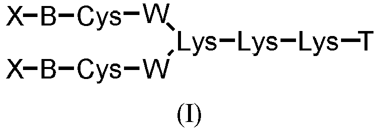

## Description

### FIELD OF THE INVENTION

The invention relates to dendrimeric peptide constructs based on a lysine core that comprise both B and T epitopes of foot-and-mouth disease virus (FMDV). The invention also refers to a process for preparing these constructs, to veterinary compositions comprising the same and to their use in the prevention of foot-and-mouth disease (FMD) in an animal.

### BACKGROUND OF THE INVENTION

Foot-and-mouth disease (FMD) is a highly contagious disease affecting cloven-hoofed animals that is caused by a virus belonging *Picornaviridae* family: FMD virus (FMDV).

Although the mortality rate is low, FMD is feared in farm industry and animal health since it is highly infectious and in case of an outbreak, massive culling of infected or suspected animals is carried out, causing a fatal economic impact. Its containment demands considerable efforts in vaccination, strict monitoring, trade restrictions and quarantines, and occasionally the elimination of millions of animals.

The FMDV particle contains a positive-strand RNA molecule of approximately 8500 nucleotides, enclosed within an icosahedral capsid comprising 60 copies of each of the four viral proteins, VP1-4. The genomic RNA encodes a single polyprotein from which the different viral proteins are generated, through a proteolitic process, due to the action of different viral proteases, which include nine mature non-structural proteins (NSPs). Each of these NSPs, as well as some of the precursor polypeptides, are involved in functions relevant to the life cycle multiplication of a virus in infected cells. FMDV shows a high genetic and antigenic variability, which is reflected in the existence of the seven serotypes (O, A, C, SAT1, SAT2, SAT3 or ASIA1) and the numerous variants.

On the other hand, the antigenic structure of the FMDV, as recognized by B lymphocytes, has been characterized in detail. The main epitope B, which is considered to be the virus' main immunogen, is continuous and immunodominant (Site A) and is located in the GH loop, around positions 140 to 160 of protein VP1 (e.g. Bittle et al, Nature 1982,298, 30-33). This epitope has been widely used as an immunogen in various studies (e.g. Brown F., Vaccine 1992, 10(14), 1022-1026). In 1986, DiMarchi and colleagues (DiMarchi et al., Science 1986, 232, 639-641) reported protection against viral challenge infection in cattle immunised with a peptide in which the VP1 residues 140-160 were colinearly synthesised with the residues corresponding to positions 200-213 of the same protein. However, further studies involving a significant number of animals have pointed out that this peptide epitope provides limited protection in natural FMDV hosts (e.g. Barteling S.J, Adv. Biotechnol. Processes 1988, 10, 25-60).

Furthermore, several T-cell epitopes recognised by lymphocytes from FMDV infected pigs have been identified in non-structural proteins (e.g. Blanco E. et al., Journal of Virology 2001, 75, 3164-3174). Among these epitopes, epitope T of protein 3A [21-35], synthesised colinearly with epitope B of VP1 [137-156], was able to efficiently stimulate lymphocytes from an infected animal and induced significant levels of serotype-specific anti-FMDV activity in vitro.

On one hand, the problem with the foot-and-mouth disease virus, which has prompted such health concerns, is that it is very contagious. The infected animals release the virus through their saliva (which is produced in abundance as a result of the disease), their urine and faeces: when the blisters burst, the virus is also released from inside. Milk and meat contain the virus, too. This means that anything which is contact with the infected animal will be easily contaminated. Likewise, the virus is very resistant under particular conditions. Therefore, it is necessary to take strict precautionary measures to avoid the contagion and spread of this illness which, although not being dangerous for human health, can cause severe economic losses.

Currently, foot-and-mouth disease is mainly controlled through the use of vaccines based on the inactivated virus. Nevertheless, several drawbacks associated with these vaccines have led FMD-free countries to follow non-vaccination policies, increasing the risk of disease reintroduction and severe outbreaks (Grubman and Baxt, Clin Microbiol Rev 2004, 17, 465-493). Therefore, the development of safer and effective vaccines is a major priority for FMD control including those based on viral subunits (Parida, Expert Rev Vaccines 2009, 8, 347-365). Targeting capsid protein VP1 for the induction of neutralizing anti-FMDV antibodies was one of the first attempts to produce peptide-based subunit vaccines. The first attempts to produce vaccines based on synthetic VP1 capsid protein were reported in the early 80s (Kleid et al., Science 1981, 214, 1125-1129), however later reports evidenced the low immunogenicity of VP1, probably due to non-native folding when expressed in a non-capsid protein context. Since then, peptides corresponding to the G-H loop in VP1 have been used as the main component of FMD peptide vaccines (Bittle et al., Nature 1982, 298, 30-33; DiMarchi et al., Science 1986, 232, 639-641; Wang et al., Vaccine 2002, 20, 2603-2610). An important advantage of this B-cell epitope is that is structurally continuous and easy to mimic as a peptide.

Despite the vaccine potential of FMDV peptides, the main limitation faced during decades was their weak immunogenicity when compared with conventional vaccines that use inactivated virus as immunogen, a limitation that may lead to selection of antigenic variants in partially immunized animals (Taboga et al., J Virol 1997, 71, 2606-2614). Optimization of the B-cell sites and inclusion in peptide vaccines of specific T-cell epitopes recognized by different MHC molecules capable of evoking adequate T-cell responses, are requirements for optimal production of FMDV neutralizing antibodies (nAbs) and have therefore been included in the composition of linear vaccine peptides (Collen, 1994, Foot-and-mouth disease virus (aphthovirus): viral T cell epitopes, In: Goddeevis, B.M.L., Morrison, I. (Eds.) Cell mediated immunity in ruminants. CRC Press Inc, Boca Raton, USA, 173-197; Cubillos et al., Virol J 2012, 9, 66; Sobrino et al., Dev Biol Stand 1999, 101, 39-43). Nevertheless, since classical linear peptides barely achieved levels of protection in livestock as those required for their use as commercial vaccines (Rodriguez et al., Vaccine 2003, 21, 3751-3756; Taboga et al., J Virol 1997, 71, 2606-2614), multimerization strategies have been developed to overcome this low-immunogenicity. One of this approaches is the so-called multiple antigenic peptides (MAPs), in which, using a single scaffold molecule, the B-cell epitope is branched from a core of lysines resulting in a dendrimer peptide (Tarn, Proc Natl Acad Sci USA 1988, 85, 5409-5413). Interestingly, two doses (2 mg each) of a dendrimer peptide displaying four copies of the G-H loop from a type C FMDV linked to a heterotypic and highly conserved T-cell epitope from FMDV 3A protein [3A (21-35)], was able to protect pigs against homologous FMDV challenge (Cubillos et al., J Virol 2008, 82, 7223-7230). Remarkably, downsized versions bearing two copies of the B-cell epitope afforded full protection in swine against an epidemiologically relevant type O FMDV even upon a single peptide dose (Blanco et al., Antiviral Res 2016, 129, 74-80; Canas-Arranz et al., 2020, A Single Dose of Dendrimer B2T Peptide Vaccine Partially Protects Pigs against Foot-and-Mouth Disease Virus Infection. Vaccines (Basel) 8).

The protective responses elicited by peptide dendrimers containing two epitopes B and one epitope T of the non-structural protein 3A of the FMDV (B₂T-3A) and other related dendrimeric constructs (hereafter B₂T-dendrimers), associates with the induction of high titers of nAbs and the activation of specific lymphocytes that would provide T-cell help for effective production of nAbs (Blanco et al., Antiviral Res 2016, 129, 74-80; Cubillos et al., J Virol 2008, 82, 7223-7230). Besides, such T-cell epitopes can also stimulate T-cell subsets leading to the expression of IFN-γ, a cytokine with a relevant role in the antiviral response (McCullough and Sobrino, 2004, Immunology of Foot-and-mouth disease, In: Sobrino, F., Domingo, E. (Eds.) Foot and mouth disease: current perspectives. Horizon Bioscience, Norfolk.). However, the MHC restriction phenomenon can limit the recognition by T-cells of B₂T-3A and related peptide dendrimers among different pig individuals as well as between FMDV host species (Collen and Doel, J Gen Virol 1990, 71(Pt 2), 309-315; Garcia-Briones et al., Vaccine 2000, 19, 1167-1171).

Therefore, further FMD vaccines are still needed. Specially, systems which elicit high levels of neutralizing antibodies and a potent response of IFN-γ producing-cells would be desirable.

### SUMMARY OF THE INVENTION

The inventors have discovered a dendrimeric construct of the B₂T-type which surprisingly elicits high titers of cross-neutralizing antibodies. In addition, this construct induces levels of cells producing IFN-γ, a potent antiviral, higher than those elicited by previous related constructs, such as B₂T3A in response to either the whole homologous dendrimer or the specific T-cell epitope. This result support that T-3D epitope, in the context of a B2T-dendrimer, is a more potent inducer of IFN-γ-producing cells compared to T-3A epitope, leading to the possibility of using T-3D to enhance the immunogenicity and the protection conferred by B₂T-dendrimers. This finding also extends the repertoire of FMDV T-cell epitopes efficiently recognized by swine lymphocytes, which can be used to enhance the immunogenicity and the protection conferred by B₂T-dendrimer constructs, as well as to cope with the potential variability in the host response to these type of dendrimer constructs due to the MHC allele diversity of populations of FMDV natural hosts (E. Blanco, D. Andreu and F. Sobrino. Peptide vaccines against foot-and-mouth disease virus. In: Foot and mouth disease virus: current research and emerging trends. pp.317-332. F. Sobrino and E. Domigo (eds.). doi.org/10.21775/9781910190517.15. Caister Academic Press, Norfolk, UK (2017); Hammer, et al., Annu Rev Anim Biosci, 2020, 8, 171-198.).

In the first aspect, the present invention relates to a dendrimeric peptide construct of formula (I) 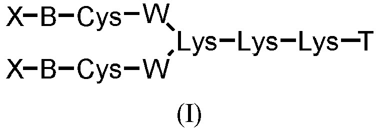 wherein
T is a T epitope of the non-structural protein 3D of the FMDV, bound to the lysine core by its N-terminal end;
B is a B epitope of the FMDV;
W is a linker group; and
X is an acyl (preferably acetyl) group when B is bound to the α-amino group of the Cys by its C-terminal end, or is a -NH₂ group or is -OH when B is bound to the α-carboxylic group of the Cys by its N-terminal end.

In the second aspect, the present invention relates to a veterinary composition comprising a dendrimeric peptide construct of formula (I) as defined in the first aspect and a veterinary acceptable excipient.

In the third aspect, the present invention relates to a dendrimeric peptide construct of formula (I) as defined in the first aspect for use in medicine.

In the fourth aspect, the present invention relates to a dendrimeric peptide construct of formula (I) as defined in the first aspect for use in the prevention of foot-and-mouth disease in an animal.

### BRIEF DESCRIPTION OF THE FIGURES

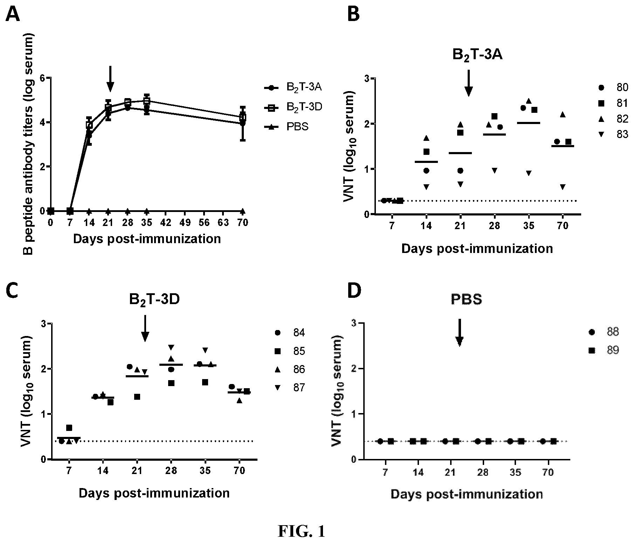

**Figure 1****.** Antibody responses of peptides B₂T-3D (example) and B₂T-3A (comparative example). (A) Total IgG specific antibody titers measured by ELISA in sera collected at different days pi. Points depict mean antibody titers for each group of pigs. VNT (virus neutralization test) in sera from animals immunized with (B) B₂T-3A, (C) B₂T-3D and (D) non-immunized. Titers are expressed as the reciprocal log₁₀ of the last serum dilution that neutralized 100 TCID50 of homologous FMDV. Each symbol represents the value for an individual pig. Horizontal lines indicate the geometric mean for each animal group (n=4) and dotted lines the detection limit. The arrows show the day of the boost.

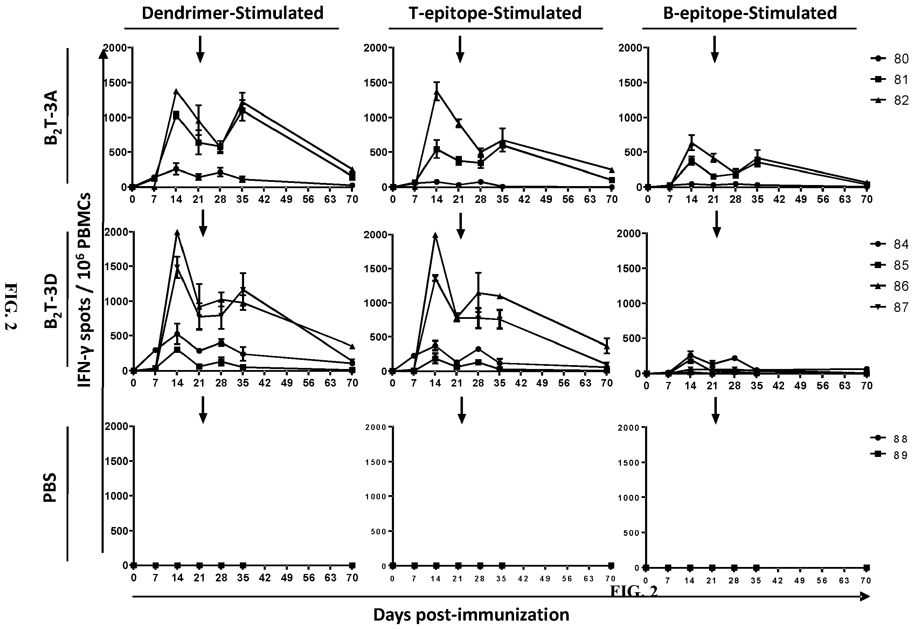

**Figure 2****.** T-cell responses in pigs immunized with B₂T-3A (comparative example) and B₂T-3D (example) constructions. PBMCs isolated from individual animals of each group were collected at different days pi. Cells were stimulated *in vitro* for 48 h with homologous dendrimer, T-cell epitope or B-cell epitope, and the number of cells expressing IFN-γ was measured by ELISPOT. PBMCs stimulated with medium (not shown) were included as a negative control of the assay and subtracted. Each point represents the mean of a triplicate of an individual animal. Arrows show the day of the boost.

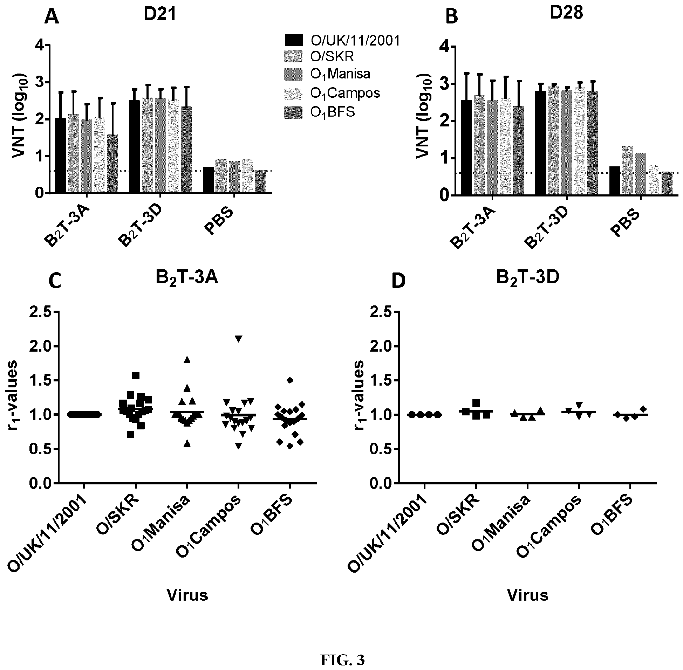

**Figure 3****.** Effect of sera from pigs immunized with B₂T-dendrimers in a panel of different FMDVs type O topotypes. Sera recovered from animals immunized with B₂T-3A (comparative example) and B₂T-3D (example) at days A) 21 and B) 28 pi were tested for its capability to neutralize a panel of different type O FMDVs. Individual columns represent the mean of each group (n=4) ± SD. Values are expressed as the reciprocal log₁₀ of the last serum dilution that neutralized 100 TCID50 of each FMDV. C and D) Antigenic relationship (*r₁*) values of the six viruses. The serological match (r₁-values; calculated as described in Materials and Methods) of sera from pigs immunized with B₂T-3A including those from previous experiments. C) and B₂T-3D D) is shown and each symbol represents the value for an individual pig. Horizontal lines indicate the geometric mean for each animal group against each virus (n=18 for B₂T-3A and n=4 for B₂T-3D).

### DETAILED DESCRIPTION OF THE INVENTION

### Dendrimeric peptide constructs

The first aspect of the present invention relates to a dendrimeric peptide construct of formula (I) 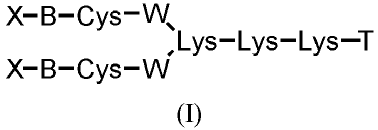 wherein
T is a T epitope of the non-structural protein 3D of the FMDV, bound to the lysine core by its N-terminal end;
B is a B epitope of the FMDV;
W is a linker group; and
X is an acyl group when B is bound to the α-amino group of the Cys by its C-terminal end, or either is a -NH₂ group or is -OH when B is bound to the α-carboxylic group of the Cys by its N-terminal end.

The core of lysines represented by 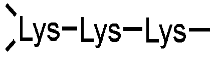 corresponds to the following chemical structure: 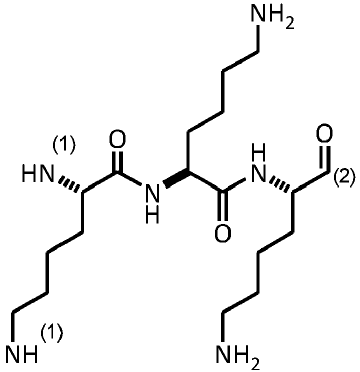 wherein the amino groups marked with (1) are linked to the linker group W and the carbonyl group marked with (2) forms part of the peptidic bond linking with epitope T. T epitope 3D of the FMDV is well known in the art (e.g. Garcia-Briones et al., Virology 2004, 322, 264-275), or can be identified empirically (e.g. using PEPSCAN or similar methods, Geysen et al. (1984) PNAS USA 81:3998, Carter (1994) Methods Mol Biol 36:207), or can be predicted (International Journal of Biotechnology Applications 2009, 1:1-3; Journal of General Virology 2011, 2297; BMC Bioinformatics 2009, 10:302). In particular, the T epitope of the 3D protein of FMDV may be identified using in vitro T-cell stimulation techniques of component proteins, protein fragments and peptides to identify appropriate sequences (Ann. Rev. Immunol., 1, 465, (1983); "The Year in Immunology, vol. 2", 1986, p. 151; Ann. Rev. Immunol., 5, 477, (1987)).

In a preferred embodiment, T is a T epitope of the 3D protein of the FMDV comprising residues 56-70 of protein 3D of FMDV serotype C (in particular C-S8; Garcia-Briones et al. Vaccine 2000, 19, 1167-1171), or the corresponding amino acid residues of another serotype of FMDV, such as serotypes O, A, SAT1, SAT2, SAT3 or ASIA1.

In a particular embodiment, T is a T epitope of the 3D protein of the FMDV consisting of residues 56-70 of protein 3D of FMDV serotype C (in particular C-S8; Garcia-Briones et al. Vaccine 2000, 19, 1167-1171), or the corresponding amino acid residues of another serotype of FMDV, such as serotypes O, A, SAT1, SAT2, SAT3 or ASIA1.

In a particular embodiment, T epitope of 3D protein of FMDV is a T epitope of 3D protein of FMDV selected from serotypes C and O, preferably of FMDV selected from serotype C strain C-S8 and serotype O strain UKG 11/01. In a preferred embodiment, T epitope of 3D protein of FMDV is a T epitope of 3D protein of FMDV serotypes C, more preferably serotype C strain C-S8.

In a more preferred embodiment, T comprises the amino acid sequence:
IFSKH-AA₁-G-AA₂-AA₃-KM-AA₄-AA₅-AA₆-D
(SEQ ID NO: 1)
wherein AA₁ is an amino acid selected from R (arginine, Arg) and K (lysine, Lys), AA₂ is amino acid selected from D (aspartic acid, Asp) and N (asparagine, Asn), AA₃ is an amino acid selected from T (threonine, Thr) and A (alanine, Ala), AA₄ is an amino acid selected from S (serine, Ser) and T (threonine, Thr), AA₅ is an amino acid selected from A (alanine, Ala), E (glutamic acid, Glu), P (proline, Pro) and K (lysine, Lys), and AA₆ is an amino acid selected from E (glutamic acid, Glu) and A (alanine, Ala).

In a particular embodiment, T consists of the amino acid sequence:
IFSKH-AA₁-G-AA₂-AA₃-KM-AA₄-AA₅-AA₆-D
(SEQ ID NO: 1)
wherein AA₁ is an amino acid selected from R and K, AA₂ is amino acid selected from D and N, AA₃ is an amino acid selected from T and A, AA₄ is an amino acid selected from S and T, AA₅ is an amino acid selected from A, E, P and K, and AA₆ is an amino acid selected from E and A.

In a more preferred embodiment, T comprises the amino acid sequence:
IFSKH-AA₁-GDTKM-AA₄-A-AA₆-D
(SEQ ID NO: 2)
wherein AA₁ is an amino acid selected from R and K, AA₄ is an amino acid selected from S and T, and AA₆ is an amino acid selected from E and A.

In a particular embodiment, T consists of the amino acid sequence:
IFSKH-AA₁-GDTKM-AA₄-A-AA₆-D
(SEQ ID NO: 2)
wherein AA₁ is an amino acid selected from R and K, AA₄ is an amino acid selected from S and T, and AA₆ is an amino acid selected from E and A.

In a particular embodiment, T comprises an amino acid sequence selected from the group consisting of IFSKHRGDTKMSAED (SEQ ID NO:3), IFSKHKGDTKMSAED (SEQ ID NO: 7), IFSKHKGNTKMSEED (SEQ ID NO: 8), IFSKHKGDTKMSEED (SEQ ID NO: 9), IFSKHKGDTKMTPED (SEQ ID NO: 10), IFSKHKGDTKMSKED (SEQ ID NO: 11), IFSKHKGDAKMSEAD (SEQ ID NO: 12) and IFSKHKGDTKMTEED (SEQ ID NO: 13).

In a particular embodiment, T consists of an amino acid sequence selected from the group consisting of IFSKHRGDTKMSAED (SEQ ID NO:3), IFSKHKGDTKMSAED (SEQ ID NO: 7), IFSKHKGNTKMSEED (SEQ ID NO: 8), IFSKHKGDTKMSEED (SEQ ID NO: 9), IFSKHKGDTKMTPED (SEQ ID NO: 10), IFSKHKGDTKMSKED (SEQ ID NO: 11), IFSKHKGDAKMSEAD (SEQ ID NO: 12) and IFSKHKGDTKMTEED (SEQ ID NO: 13).

In a still more preferred embodiment, T comprises the amino acid sequence IFSKHRGDTKMSAED (SEQ ID NO: 3).

In still more preferred embodiment, T consists of the amino acid sequence IFSKHRGDTKMSAED (SEQ ID NO: 3).

Preferably, the T epitope is capped at the C-terminus as a carboxamide.

B epitopes of the FMDV are well known in the art (e.g. Veterinary Research 2001, 32:1-30; Mateu, M.G. 17. The Foot-and-mouth Disease Virion: Structure and Function, In: Sobrino, F., Domingo, E. (Eds.) Foot-and-mouth Disease Virus. Current Research and Emerging Trends. Caister Academic Press, Norfolk, UK, 61-105), or they can be identified empirically (e.g. using PEPSCAN or similar methods, Geysen et al. (1984) PNAS USA 81:3998, Carter (1994) Methods Mol Biol 36:207), or can be predicted (International Journal of Biotechnology Applications 2009, 1:1-3; Journal of General Virology 2011, 2297; BMC Bioinformatics 2009, 10:302).

In a preferred embodiment, B is a B epitope of the capsid protein VP1, VP2 or VP3 of the FMDV, preferably a B epitope of the capsid protein VP1.

The main B epitope of FMDV has been identified as a region called GH loop or antigenic site, which is located approximately between residues 140-160 of VP1.

In a preferred embodiment, B is a B epitope of the FMDV comprising and antigenic site of protein VP1.

The skilled person can identify an antigenic site of protein VP1 using routine experimentation, for example by empirical methods (e.g. using peptides: Strohmaier, K., Franze, R., Adam, K.H., J Gen Virol, 1982, 59, 295-306., PEPSCAN or similar methods, Geysen et al. PNAS USA, 1984, 81:3998, Carter Methods Mol Biol, 1994, 36:207), in silico predictions (Maree F.F. et al., Journal of General Virology, 2011, 92, 2297-2309; Liang S. et al., BMC Bioinformatics, 2009, 10:302), or neutralizing antibodies (Mateu, M.G. 2017. The Foot-and-mouth Disease Virion: Structure and Function, In: Sobrino, F., Domingo, E. (Eds.) Foot-and-mouth Disease Virus. Current Research and Emerging Trends. Caister Academic Press, Norfolk, UK, 61-105).

In a preferred embodiment, B is a B epitope of the FMDV comprising residues 140-157 of protein VP1 of FMDV serotype O (O-UKG 11/01, O1Campos, O1BFS strains); even more preferably B is a B epitope of the FMDV comprising residues 140-157 of protein VP1 of FMDV serotype O (in particular O-UKG 11/01).

In a particular embodiment, B is a B epitope of the FMDV consisting of residues 140-157 of protein VP1 of FMDV serotype O (O-UKG 11/01, O1Campos, O1BFS strains); even more preferably B is a B epitope of the FMDV consisting of residues 140-157 of protein VP1 of FMDV serotype O (in particular O-UKG 11/01).

In a particular embodiment, B epitope of FMDV is a B epitope of FMDV serotype O, preferably FMDV serotype O-UKG 11/01.

In a preferred embodiment, B epitope comprises the amino acid sequence:
AA₇-AA₈-AA₉-AA₁₀-AA₁₁-RGDLQVLAQKAART
(SEQ ID NO: 4)
wherein AA₇ is an amino acid selected from P (proline, Pro), S (serine, Ser) and A (alanine, Ala), AA₈ is an amino acid selected from V (valine, Val), M (methionine, Met), A (alanine, Ala), T (threonine, Thr) and E (glutamic acid, Glu), AA₉ is an amino acid selected from T (threonine, Thr) and A (alanine, Ala), AA₁₀ is an amino acid selected from N (asparagine, Asn) and S (serine, Ser), and AA₁₁ is an amino acid selected from V (valine, Val) and L (leucine, Leu).

In a particular embodiment, B epitope consists of the amino acid sequence:
AA₇-AA₈-AA₉-AA₁₀-AA₁₁-RGDLQVLAQKAART
(SEQ ID NO: 4)
wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L.

In a particular embodiment, B epitope comprises an amino acid sequence selected from the group consisting of PVTNVRGDLQVLAQKAART (SEQ ID NO: 14), TVANVRGDLQVLAQKAARA (SEQ ID NO: 15), PTTNVRGDLQVLAQKAART (SEQ ID NO: 16), PTTNVRGDLQVLAQKAARM (SEQ ID NO: 17), AVPNVRGDLQVLAQKVVRT (SEQ ID NO: 18) and AVPNLRGDLQVLAQKVART (SEQ ID NO: 19).

In a particular embodiment, B epitope consists of an amino acid sequence selected from the group consisting of PVTNVRGDLQVLAQKAART (SEQ ID NO: 14), TVANVRGDLQVLAQKAARA (SEQ ID NO: 15), PTTNVRGDLQVLAQKAART (SEQ ID NO: 16), PTTNVRGDLQVLAQKAARM (SEQ ID NO: 17), AVPNVRGDLQVLAQKVVRT (SEQ ID NO: 18) and AVPNLRGDLQVLAQKVART (SEQ ID NO: 19).

In a more preferred embodiment, B epitope comprises the amino acid sequence PVTNVRGDLQVLAQKAART (SEQ ID NO: 5).

In a still more preferred embodiment, B consists of amino acid sequence PVTNVRGDLQVLAQKAART (SEQ ID NO: 5).

In a particular embodiment, T epitope of 3D protein of FMVD is an epitope of FMVD serotype C and the B epitope of FMVD is an epitope of FMVD serotype O.

T and B epitopes of the FMDV include peptides that, compared to the sequences disclosed herein, include one or more (e.g. 1, 2, 3, 4, 5, 6, 7, 8, 9, 10, etc.) conservative amino acid substitutions i.e. replacements of the amino acid with another which has a related side chain. Such substitutions and modifications are generally well-known to those skilled in the art of peptide chemistry. Genetically-encoded amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine). In general, substitution of single amino acids within these families does not have a major effect on the biological activity. Moreover, the peptides may have one or more (e.g. 1, 2, 3, 4, 5, 6, 7, 8, 9, 10, etc.) single amino acid deletions relative to the sequences disclosed herein. Furthermore, the peptides may include one or more (e.g. 1, 2, 3, 4, 5, 6, 7, 8, 9, 10, etc.) insertions relative to the sequences disclosed herein.

In a preferred embodiment, in the dendrimeric construct of formula (I)
B comprises the amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T comprises the amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A.

In a preferred embodiment, in the dendrimeric construct of formula (I),
B consists of amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T consists of amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A.

In a preferred embodiment, in the dendrimeric construct of formula (I), B comprises the amino acid sequence PVTNVRGDLQVLAQKAART (SEQ ID NO: 5), and T comprises the amino acid sequence IFSKHRGDTKMSAED (SEQ ID NO: 3).

In another embodiment, in the dendrimeric construct of formula (I), B consists of the amino acid sequence PVTNVRGDLQVLAQKAART (SEQ ID NO: 5), and T consists of the amino acid sequence IFSKHRGDTKMSAED (SEQ ID NO: 3).

Epitope B is bound to X, which is an acyl group when B is bound to the α-amino group of the Cys by its C-terminal end, or is either a -NH₂ group or is -OH when B is bound to the α-carboxylic group of the Cys by its N-terminal end.

In a preferred embodiment, B is bound to the α-amino group of the Cys by its C-terminal end. Thus, X is an acyl group.

The term "acyl" as used herein, refers to a group of formula R-CO- wherein R is an alkyl group having 1 to 6 carbon atoms, preferably 1 to 3 carbon atoms. More preferably, X is an acetyl group (abbreviated as Ac).

In a particular embodiment, B is bound to the α-carboxylic group of the Cys by its N-terminal end and X is a -NH₂ group, i.e. the B epitope is capped at the C-terminus as a carboxamide.

In the dendrimeric constructs of the present invention W is a linker group. The term "linker group" refers to a bi-functional unit (biradical) that makes possible to link covalently the sulphydryl of the cysteine (Cys) with an amino group of the lysine core.

In a particular embodiment, W is an organic group having from 1 to 20, preferably from 1 to 10, carbon atoms and optionally containing at least one, preferably 1, 2, 3 or 4, heteroatoms selected from N, O and S. Preferably, W is a cyclic or acyclic hydrocarbon group having from 1 to 20, preferably from 1 to 10, carbon atoms wherein optionally one or more, preferably 1, 2, 3 or 4, -CH₂- or -CH- groups are replaced by -CO-, -S-, -O-, - NH- or

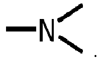

In a preferred embodiment W is selected from the group consisting of: 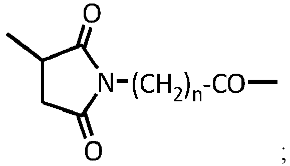 -(CH₂)ₙ-CO-; and
-CO-(CH₂)ₙ-S-(CH₂)ₘ-CO-;
wherein n and m are independently selected from 1, 2, 3, 4, 5 and 6; preferably n and m are independently selected from 1, 2 and 3; more preferably n and m are 2.

In a preferred embodiment, W is 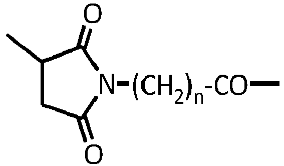 wherein n is selected from 1, 2, and 3, preferably 2.

Cys is bound to the linker group W through the -SH group and Lys may be bound to the linker group W through the ε-amino group or α-amino group. In particular, in the dendrimeric construct of the present invention, the Lys is bound to one of linker groups W through the ε amino group of Lys and to the other one of the linker groups W through the α-amino group of Lys.

In a preferred embodiment, the dendrimeric construct of the present invention has formula (Ia): 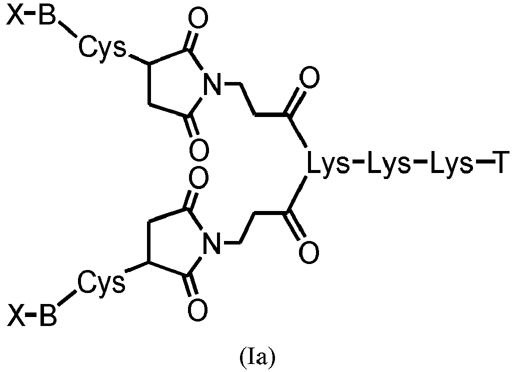 wherein X is an acyl group when B is bound to the α-amino group of the Cys by its C-terminal end, or is a -NH₂ group when B is bound to the α-carboxylic group of the Cys by its N-terminal end, and
wherein B and T are as defined above for formula (I).

In a preferred embodiment of formula (Ia), T is a T epitope of the FMDV comprising residues 56-70 of protein 3D of FMDV serotype C (in particular C-S8; Garcia-Briones et al. Vaccine 2000, 19, 1167-1171), or the corresponding amino acid residues of another serotype of FMDV, such as serotypes O, A, SAT1, SAT2, SAT3 or ASIA1.

In a particular embodiment of formula (Ia), T is a T epitope of the FMDV consisting of residues 56-70 of protein 3D of FMDV serotype C (in particular C-S8; Garcia-Briones et al. Vaccine 2000, 19, 1167-1171), or the corresponding amino acid residues of another serotype of FMDV, such as serotypes O, A, SAT1, SAT2, SAT3 or ASIA1.

In a particular embodiment, T epitope of 3D protein of FMDV is a T epitope of 3D protein of FMDV selected from serotypes C and O, preferably of FMDV selected from serotype C strain C-S8 and serotype O strain UKG 11/01. In a preferred embodiment, T epitope of 3D protein of FMDV is a T epitope of 3D protein of FMDV serotypes C, more preferably serotype C strain C-S8.

In a more preferred embodiment of formula (Ia), T comprises the amino acid sequence:
IFSKH-AA₁-G-AA₂-AA₃-KM-AA₄-AA₅-AA₆-D
(SEQ ID NO: 1)
wherein AA₁ is an amino acid selected form R and K, AA₂ is amino acid selected from D and N, AA₃ is an amino acid selected from T and A, AA₄ is an amino acid selected from S and T, AA₅ is an amino acid selected from A, E, P and K, and AA₆ is an amino acid selected from E and A.

In a particular embodiment of formula (Ia), T consists of the amino acid sequence:
IFSKH-AA₁-G-AA₂-AA₃-KM-AA₄-AA₅-AA₆-D
(SEQ ID NO: 1)
wherein AA₁ is an amino acid selected from R and K, AA₂ is amino acid selected from D and N, AA₃ is an amino acid selected from T and A, AA₄ is an amino acid selected from S and T, AA₅ is an amino acid selected from A, E, P and K, and AA₆ is an amino acid selected from E and A.

In a more preferred embodiment of formula (Ia), T comprises the amino acid sequence:
IFSKH-AA₁-GDTKM-AA₄-A-AA₆-D
(SEQ ID NO: 2)
wherein AA₁ is an amino acid selected form R and K, AA₄ is an amino acid selected from S and T, and AA₆ is an amino acid selected from E and A.

In a particular embodiment of formula (Ia), T consist of the amino acid sequence:
IFSKH-AA₁-GDTKM-AA₄-A-AA₆-D
(SEQ ID NO: 2)
wherein AA₁ is an amino acid selected form R and K, AA₄ is an amino acid selected from S and T, and AA₆ is an amino acid selected from E and A.

In a particular embodiment of formula (Ia), T comprises an amino acid sequence selected from the group consisting of IFSKHRGDTKMSAED (SEQ ID NO:3), IFSKHKGDTKMSAED (SEQ ID NO: 7), IFSKHKGNTKMSEED (SEQ ID NO: 8), IFSKHKGDTKMSEED (SEQ ID NO: 9), IFSKHKGDTKMTPED (SEQ ID NO: 10), IFSKHKGDTKMSKED (SEQ ID NO: 11), IFSKHKGDAKMSEAD (SEQ ID NO: 12) and IFSKHKGDTKMTEED (SEQ ID NO: 13).

In a particular embodiment of formula (Ia), T consists of an amino acid sequence selected from the group consisting of IFSKHRGDTKMSAED (SEQ ID NO:3), IFSKHKGDTKMSAED (SEQ ID NO: 7), IFSKHKGNTKMSEED (SEQ ID NO: 8), IFSKHKGDTKMSEED (SEQ ID NO: 9), IFSKHKGDTKMTPED (SEQ ID NO: 10), IFSKHKGDTKMSKED (SEQ ID NO: 11), IFSKHKGDAKMSEAD (SEQ ID NO: 12) and IFSKHKGDTKMTEED (SEQ ID NO: 13).

In a still more preferred embodiment of formula (Ia), T comprises the amino acid sequence IFSKHRGDTKMSAED (SEQ ID NO: 3).

In still more preferred embodiment of formula (Ia), T consists of the amino acid sequence IFSKHRGDTKMSAED (SEQ ID NO: 3).

Preferably, the T epitope in formula (Ia) is capped at the C-terminus as a carboxamide.

As previously explained, B epitopes of the FMDV are well known in the art (Mateu, M.G. 2017. The Foot-and-mouth Disease Virion: Structure and Function, In: Sobrino, F., Domingo, E. (Eds.) Foot-and-mouth Disease Virus. Current Research and Emerging Trends. Caister Academic Press, Norfolk, UK, 61-105), or they can be identified empirically (e.g. using PEPSCAN or similar methods, Geysen et al., PNAS USA, 1984, 81:3998, Carter, Methods Mol Biol, 1994, 36:207), or can be predicted (Maree F.F. et al., Journal of General Virology, 2011, 92, 2297-2309; Liang S. et al., BMC Bioinformatics 2009, 10:302).

In a preferred embodiment of formula (Ia), B is a B epitope of the capsid protein VP1, VP2 or VP3 of the FMDV.

In a preferred embodiment of formula (Ia), B is a B epitope of the FMDV comprising and antigenic site of protein VP1.

The skilled person can identify an antigenic site of protein VP1 using routine experimentation, for example by using empirical methods (e.g. using peptides: Strohmaier, K., Franze, R., Adam, K.H., 1982. Location and characterization of the antigenic portion of the FMDV immunizing protein. J Gen Virol 59, 295-306., PEPSCAN or similar methods, Geysen et al., PNAS USA, 1984, 81:3998, Carter, Methods Mol Biol, 1994 36:207), in silico predictions (Maree F.F. et al., Journal of General Virology, 2011, 92, 2297-2309; Liang S. et al., BMC Bioinformatics, 2009, 10:302), or neutralizing antibodies (Mateu, M.G. 2017. The Foot-and-mouth Disease Virion: Structure and Function, In: Sobrino, F., Domingo, E. (Eds.) Foot-and-mouth Disease Virus. Current Research and Emerging Trends. Caister Academic Press, Norfolk, UK, 61-105).

In a preferred embodiment of formula (Ia), B is a B epitope of the FMDV comprising residues 140-157 of protein VP1 of FMDV serotype O (O-UKG 11/01, O1Campos, O1BFS strains); even more preferably B is a B epitope of the FMDV comprising residues 140-157 of protein VP1 of FMDV serotype O (preferably O-UKG 11/01).

In a particular embodiment, B is a B epitope of the FMVD consisting of residues 140-157 of protein VP1 of FMDV serotype O (O-UKG 11/01, O1Campos, O1BFS strains); even more preferably B is a B epitope of the FMDV consisting of residues 140-157 of protein VP1 of FMDV serotype O (preferably O-UKG 11/01).

In a particular embodiment, B epitope of FMDV is a B epitope of FMDV serotype O, preferably FMDV serotype O strain UKG 11/01.

In a preferred embodiment of formula (Ia), B epitope comprises the amino acid sequence:
AA₇-AA₈-AA₉-AA₁₀-AA₁₁-RGDLQVLAQKAART
(SEQ ID NO: 4)
wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L.

In a particular embodiment of formula (Ia), B epitope consists of the amino acid sequence:
AA₇-AA₈-AA₉-AA₁₀-AA₁₁-RGDLQVLAQKAART
(SEQ ID NO: 4)
wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L.

In a particular embodiment of formula (Ia), B epitope comprises an amino acid sequence selected from the group consisting of PVTNVRGDLQVLAQKAART (SEQ ID NO: 14), TVANVRGDLQVLAQKAARA (SEQ ID NO: 15), PTTNVRGDLQVLAQKAART (SEQ ID NO: 16), PTTNVRGDLQVLAQKAARM (SEQ ID NO: 17), AVPNVRGDLQVLAQKVVRT (SEQ ID NO: 18) and AVPNLRGDLQVLAQKVART (SEQ ID NO: 19).

In a particular embodiment of formula (Ia), B epitope consists of an amino acid sequence selected from the group consisting of PVTNVRGDLQVLAQKAART (SEQ ID NO: 14), TVANVRGDLQVLAQKAARA (SEQ ID NO: 15), PTTNVRGDLQVLAQKAART (SEQ ID NO: 16), PTTNVRGDLQVLAQKAARM (SEQ ID NO: 17), AVPNVRGDLQVLAQKVVRT (SEQ ID NO: 18) and AVPNLRGDLQVLAQKVART (SEQ ID NO: 19).

In a more preferred embodiment of formula (Ia), B epitope of the FMDV comprises the amino acid sequence PVTNVRGDLQVLAQKAART (SEQ ID NO: 5).

In a still more preferred embodiment of formula (Ia), B consists of amino acid sequence PVTNVRGDLQVLAQKAART (SEQ ID NO: 5).

In a particular embodiment, T epitope of 3D protein of FMVD is an epitope of FMVD serotype C and the B epitope of FMVD is an epitope of FMVD serotype O.

Epitope B is bound to X, which is an acyl group when B is bound to the α-amino group of the Cys by its C-terminal end, or is either a -NH₂ group or is -OH when B is bound to the α-carboxylic group of the Cys by its N-terminal end.

In a preferred embodiment, B is bound to the α-amino group of the Cys by its C-terminal end. Thus, X is an acyl group, preferably an acetyl group.

In another embodiment, B is bound to the α-carboxylic group of the Cys by its N-terminal end and X is a -NH₂ group, i.e. the B epitope is capped at the C-terminus as a carboxamide.

In another embodiment, B is bound to the α-carboxylic group of the Cys by its N-terminal end and X is a -OH group, i.e. the B epitope is not capped by any group at the C-terminus ant therefore terminates with the -OH group of the carboxylic acid group of the C-terminus amino acid.

In a preferred embodiment of formula (Ia), in the dendrimeric construct of formula (Ia)
B is bound to the α-amino group of the Cys by its C-terminal end,
X is an acetyl group,
B comprises the amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T comprises the amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A, wherein T is capped at the C-terminus as a carboxamide.

In another embodiment of formula (Ia), in the dendrimeric construct of formula (Ia),
B is bound to the α-carboxylic group of the Cys by its N-terminal end,
X is a -NH₂ group,
B comprises the amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T comprises the amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A, wherein T is capped at the C-terminus as a carboxamide.

In a preferred embodiment of formula (Ia), in the dendrimeric construct of formula (Ia),
B is bound to the α-amino group of the Cys by its C-terminal end,
X is an acetyl group,
B consists of the amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T consists of amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A, wherein T is capped at the C-terminus as a carboxamide.

In another embodiment of formula (Ia), in the dendrimeric construct of formula (Ia),
B is bound to the α-carboxylic group of the Cys by its N-terminal end,
X is a -NH₂ group,
B consists of the amino acid sequence AA₇-AA₈-AA₉-AA₁₀-AA₁₁-RGDLQVLAQKAART (SEQ ID NO: 4), wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L, and
T consists of amino acid sequence IFSKH-AA₁-GDTKM-AA₄-A-AA₆-D (SEQ ID NO: 2), wherein AA₁ is an amino acid selected form R and K, AA₄ is an amino acid selected from S and T, and AA₆ is an amino acid selected from E and A, wherein T is capped at the C-terminus as a carboxamide.

In a preferred embodiment of formula (Ia), in the dendrimeric construct of formula (Ia), B is bound to the α-amino group of the Cys by its C-terminal end, X is an acetyl group, B comprises the amino acid sequence PVTNVRGDLQVLAQKAART (SEQ ID NO: 5), and T comprises the amino acid sequence IFSKHRGDTKMSAED (SEQ ID NO: 3) capped at the C-terminus as a carboxamide.

In another embodiment of formula (Ia), in the dendrimeric construct of formula (Ia), B is bound to the α-carboxylic group of the Cys by its N-terminal end, X is a -NH₂ group, B comprises the amino acid sequence PVTNVRGDLQVLAQKAART (SEQ ID NO: 5), and T comprises the amino acid sequence IFSKHRGDTKMSAED (SEQ ID NO: 3) capped at the C-terminus as a carboxamide.

In a preferred embodiment of formula (Ia), in the dendrimeric construct of formula (Ia), B is bound to the α-amino group of the Cys by its C-terminal end, X is an acetyl group, B consists of the amino acid sequence PVTNVRGDLQVLAQKAART (SEQ ID NO: 5), and T consists of the amino acid sequence IFSKHRGDTKMSAED (SEQ ID NO: 3) capped at the C-terminus as a carboxamide.

In another embodiment of formula (Ia), in the dendrimeric construct of formula (Ia), B is bound to the α-carboxylic group of the Cys by its N-terminal end, X is a -NH₂ group, B consists of the amino acid sequence PVTNVRGDLQVLAQKAART (SEQ ID NO: 5), and T consists of the amino acid sequence IFSKHRGDTKMSAED (SEQ ID NO: 3) capped at the C-terminus as a carboxamide.

### Preparation of the dendrimeric peptide constructs

The present invention relates to a process for the preparation of a dendrimeric peptide construct of formula (Ia) as defined above 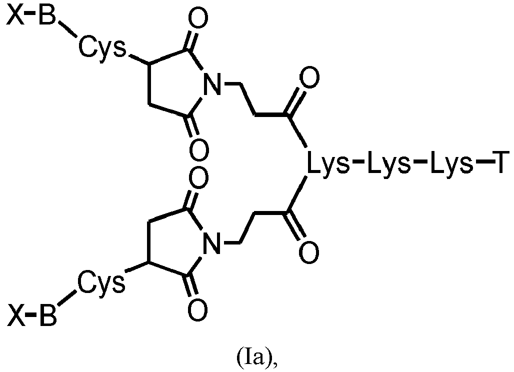 the process comprising the reaction of a compound of formula (II) 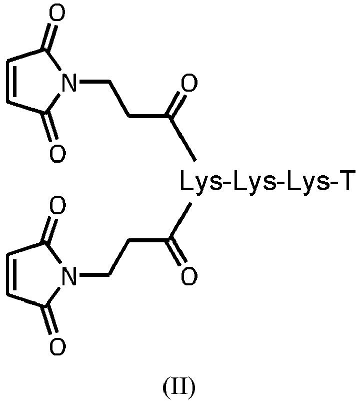 with a compound of formula (III)

X-B-Cys (III)

wherein X, B and T are as defined in above,
at a pH between 5.5 and 7.5, preferably at a pH between 5.5 and 7.0, more preferably at a pH between 5.5 and 6.5, still more preferably at a pH about 6.0.

Peptide constructs of formula (I) may also be obtained by reaction of a compound of formula (IV) 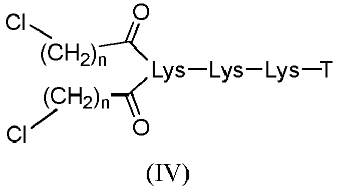 with a compound of formula (III)

X-B-Cys (III)

wherein X, B, T and n are as defined in the first aspect,
at a pH between 6.0 and 7.5.

Alternatively, constructs of formula (I) can be obtained by a process which comprises the reaction of a compound of formula (V) 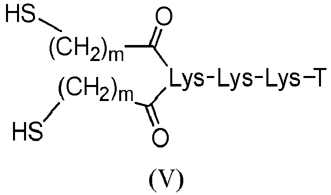 with a compound of formula (VI) 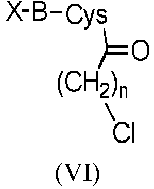 wherein X, T, B, m and n are as defined in the first aspect,
at a pH between 6.0 and 7.5.

Compounds of formula (II), (III), (IV), (V) and (VI) can be obtained by solid phase peptide synthesis.

In a particular embodiment, compound of formula (II) can be obtained by first synthesizing the T epitope on a solid support, preferably a polymeric resin, using Fmoc chemistry in an automated synthesizer, then the lysine core is introduced by coupling with Fmoc-Lys(Fmoc)-OH and then the α and ε amino groups are deprotected and functionalized with at α and ε amino groups by using N-maleoyl-β-alanine, and cleaved from the solid support.

In a particular embodiment, compound of formula (III) can be obtained by peptide synthesis on a solid support, preferably a polymeric resin, using Fmoc chemistry in an automated synthesizer. The resulting peptide can be acylated or not at the N-terminal end, then deprotected and cleaved from the solid support.

In a particular embodiment, the compound of formula (IV) can be obtained by first synthesizing the T epitope on a solid support, preferably a polymeric resin, using Fmoc chemistry in an automated synthesizer, then the lysine core is introduced by coupling with Fmoc-Lys(Fmoc)-OH and then the α and ε amino groups are deprotected and functionalized with a -W-Cl group. The resulting peptide is deprotected and cleaved from the solid support. Preferably, when W is a -(CH₂)ₙ-CO- group, the deprotected and cleaved peptide is chloroacetylated at α and ε amino groups, preferably by treatment with chloroacetic acid.

In a particular embodiment, compound of formula (V) can be obtained in a similar way as described for compound (IV) where, after coupling with Fmoc-Lys(Fmoc)-OH and deprotection, the resulting peptide is functionalized at α and ε amino groups with a -CO-(CH₂)ₘ-SH group, preferably by treatment with S-trityl-3-mercaptoalkyl acid, and cleaved from the solid support.

In a particular embodiment, compound of formula (VI) can be obtained in a similar way as described for compound (III) but wherein the C-terminal Lys is deprotected at its ε amino group, then chloroacetylated, preferably by treatment with chloroacetic acid. The resulting peptide is deprotected and cleaved from the solid support.

The term "about" is used herein to mean ±5%, preferably ±2%, more preferably ±1%, the value it characterizes.

### Compositions and uses of the dendrimeric peptide constructs

In another aspect, the invention refers to a veterinary composition that comprises at least a dendrimeric peptide construct of formula (I) as defined in the first aspect and a veterinary acceptable excipient.

Conventional excipients, in particular adjuvants, carriers and diluents may be employed. Suitable carriers and diluents are known in the art (e.g. McKercher PD et al., "A review of the current status of oil adjuvants in foot-and-mouth disease", Dev. Biol. Stand. 1976, vol. 35, p. 107-112; Dong XN et al., "Candidate multi-peptide-vaccine against classical swine fever virus induced potent immunity with serological marker", Vaccine 2005, vol. 23, p. 3630-3633) and include, for example, Freund's complete and incomplete adjuvant, aluminium hydroxide, saponin, dextrans, mineral oils, neutral oils, vegetable oils, liposomes, polyols, etc.

These compositions may be suitable as immunogenic compositions or as vaccines. The compositions can be administered by any convenient route including any oral or parenteral route, such as subcutaneous, intravenous or intramuscular.

Constructs of formula (I) have been found to present immunogenic properties. Therefore, in another aspect, the invention refers to a dendrimeric peptide construct of formula (I) for use in medicine.

In another aspect, the invention refers to a dendrimeric peptide construct of formula (I) for use in the prevention of foot-and-mouth disease in an animal.

The invention also describes the use of a dendrimeric peptide construct of formula (I) in the preparation of a medicament for the prevention of foot-and-mouth disease in an animal.

The invention also discloses a method of preventing foot-and-mouth disease in an animal comprising administering an effective amount of a dendrimeric peptide construct of formula (I).

The foot-and-mouth disease to be prevented may be caused by any FMDV. In particular, FMDV serotype O, A, C, SAT1, SAT2, SAT3 or ASIA1. Preferably, the foot-and-mouth disease is caused by FMVD serotype O. Preferably, the foot-and-mouth disease is caused by FMVD serotype O strain UKG 11/01 (O/UK/11/2001), strain SKR, strain O₁Manisa, strain O₁Campos or strain O₁BFS.

Animals to be treated include both domestic and wild animals, such as pigs, cattle, sheep, goats, buffalo, antelope, deer, wild boar, bison or any other cloven-hoofed animal, preferably pigs.

The following examples illustrate the invention and should not be considered as limitative of the invention

### EXAMPLES

### 1. Materials and Methods

### 1.1. Peptides

The B-cell epitope from FMDV O-UKG 11/01, VP1 (residues 140-157; SEQ ID NO: 5), and the T-cell epitopes 3A (residues 21-35; SEQ ID NO: 6) and 3D (residues 56-70; SEQ ID NO: 3) were synthesized by Fmoc-solid phase synthesis (SPPS), purified by reverse-phase liquid chromatography (RP-HPLC) and characterized by mass spectrometry (MS). B₂T-dendrimers were prepared by conjugation in solution of two B-cell peptides containing and additional C-terminal Cys (free thiol form) with one T-cell epitope N-terminally elongated with two Lys residues followed by an extra Lys branching point further derivatized into two maleimide groups (Table 1). The B₂T-3A and B₂T-3D constructs were obtained via thiol-maleimide ligation at pH 6.0, purified by RP-HPLC and characterized by MS (Blanco et al., Antiviral Res 2016, 129, 74-80; Cubillos et al., J Virol 2008, 82, 7223-7230; Monso et al., J Virol 2010, 82, 7223-7230).

**Table 1. B₂T bivalent dendrimeric construction. a) B₂T construct with the B epitope linked to a 3-maleimidopropionic acid unit (W) attached in the Lys core. b) Experimental peptide mass obtained by LC/MS. c) Retention time on a C18 column (Luna, 4.6 mm x 50 mm, 3 mm; Phenomenex) eluted with a 20-60% linear gradient of solvent B (0.036% TFA in MeCN) into solvent A (0.045% TFA in H₂O) over 15 min. In parenthesis, homogeneity of purified material.**

| B₂T - dendrimers | | |
|---|---|---|
| General structure^{a} | 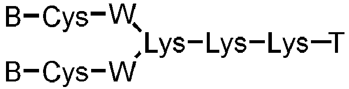 | |
| B epitope | acetyl-PVTNVRGDLQVLAQKAART -amide (acetyl-SEQ ID NO: 5-amide) | |
| T-3A | AAIEFFEGMVHDSIK-amide (SEQ ID NO: 6-amide) | |
| T-3D | IFSKHRGDTKMSAED-amide (SEQ ID NO: 3-amide) | |
| w | 3-maleimidopropionic acid unit | |
| Peptides | B₂T-3A (Comparative exemple) | B₂T-3D (Example) |
| MW^{b} | 6742.8 Da | 6770.8 Da |
| HPLC^{c} | 6.9 min (98%) | 5.1 min (98%) |

### 1.2. Viruses

The FMDV stocks O/UK/11/01, O/SKR, O₁Manisa, O₁BFS (The Pirbright Institute, UK) and O₁Campos (OPS-PanAftosa) were amplified in IBRS-2 cells.

### 1.3. Animals

White cross-bred Landrace female pigs, 9 to 12 weeks-old (20 Kg), were maintained in a conventional farm facility at the Departamento de Reproducción Animal, INIA, Madrid. Groups of four pigs were immunized with B₂T-3A or B₂T-3D at day 0 with 2 ml of Montanide ISA 50V2 emulsion containing 2 mg of the corresponding peptide and boosted at day 21 pi. Two additional pigs were PBS-inoculated and maintained as controls. Blood samples were collected at days 0, 7, 14, 21, 28, 35 and 70 pi to collect serum and peripheral blood mononuclear cells (PBMCs). The study was approved (CBS2014/015 and CEEA2014/018) by the INIA Committees on Ethics of Animal Experiments and Biosafety, and by the National Committee on Ethics and Animal Welfare (PROEX 218/14).

### 1.4. Virus neutralization test (VNT)

Neutralization assays were performed in 96-well culture plates. Serial 2-fold dilutions of each serum sample (in DMEM containing 2% fetal bovine serum) were incubated with 100 infection units - 50% tissue culture infective doses (TCID₅₀) - of FMDV O-UKG 11/01 for 1 h at 37°C. Then, a cell suspension of IBRS-2 cells in DMEM was added and plates were incubated for 72 h. Monolayers were then controlled for development of cytopathic effect (cpe), fixed at 96 h pi and stained. End-point titers were calculated as the reciprocal of the final serum dilution that neutralized 100 TCID₅₀ of homologous FMDV in 50% of the wells (Blanco et al., Antiviral Res 2016, 129, 74-80).

### 1.5. Detection of anti-FMDV antibodies by ELISA

Specific antibodies were assayed by ELISA as described (Blanco et al., Antiviral Res 1016, 129, 74-80) using plates coated with peptide B (1µg) that were incubated with 3-fold dilutions of serum and detected using HRP-conjugated protein A. Plates were read at 450 nm and titers expressed as the reciprocal of the last serum dilution given an absorbance range of two standard deviations above the background (serum at day 0) plus 2 SD. Specific IgG1 and IgG2 titers to peptide B were measured and expressed as above using isotype-specific mAbs (Serotec) that were detected with HRP-labeled anti-mouse (Thermo Fisher). Antibodies against the nonstructural protein precursor 3ABC were detected basically as described (Blanco et al., Antiviral Res 2016, 129, 74-80; Borrego et al., Antiviral Res 2017, 142, 30-36).

### 1.6. PBMC isolation and IFN-γ detection by ELISPOT

Porcine PBMCs were isolated from blood samples collected in Vacutainer tubes EDTA-K2, diluted 1:1 in PBS and then used to obtain PBMC by density-gradient centrifugation with Histopaque 1077 (Sigma) and Leucosep tubes (Greiner Bio-One) as described (Borrego et al., Antiviral Res 2017, 142, 30-36). For the IFN-γ ELISPOT assay 2.5×10⁵ PBMCs were shed in triplicate wells of Immobilon-P plates (Merck Millipore) coated as reported (Blanco et al., Antiviral Res 2016, 129, 74-80) and in vitro stimulated with 50 µg/ml of their respective immunogenic peptides. As positive or negative controls, cells were incubated with 10 µg/ml of phytohaemagglutinin (Sigma) or only with medium, respectively. After 48 h at 37°C and 5% CO₂, plates were washed and incubated with a biotinylated anti-mouse IFN-γ (clone P2C11, BD) followed by HRP-streptavidin (BD). The frequency of peptide-specific T-cells was expressed as the mean number of spot-forming cells/10⁶ PBMCs, with background values (number of spots in negative control wells) subtracted from the respective counts of stimulated cells. These experiments were performed using outbred domestic pigs with different individual genetic backgrounds. In any case, the levels of animal-to-animal variation did not exceed those observed in other related studies (Taboga et al., J Virol 1997, 71, 2606-2614).

### 1.7. Statistical analyses

Differences among peptide-immunized groups in FMDV-antibody titers and number of IFN-γ producing cells were analyzed using the Student's t-test. Values are cited in the text as mean ± SD. All *p* values are two sided, and *p* values <0.05 were considered significant. Statistical analyses were conducted using GraphPad Prism Software 5.0

### 2. Results

### 2.1. Immunogenicity in swine of a B₂T construction harboring a T-cell epitope from FMDV non-structural protein 3D

The immune response elicited by B₂T-3D and B₂T-3A in pigs was tested, including the longevity of the response. To this end, groups of four pigs were immunized with 2 mg of B₂T-3A (pigs 80, 81, 82 and 83), B₂T-3D (pigs 84, 85, 86 and 87) or non-immunized (88 and 89). At day 21 the animals were boosted with the same amount of peptide and sera and PBMCs samples were collected at the indicated times. One animal from B₂T-3A group (pig 83) showed a deteriorated health status during the second week of the experiment, being excluded from the analysis.

### 2.1.1. Dendrimers B₂T-3D and B₂T-3A elicit similar antibody responses

The total IgG antibodies elicited by the peptides were measured by ELISA. Specific antibodies were detected in both groups at day 14 pi from which a gradual increment was observed. No remarkable boost effect was observed neither in B₂T-3A nor in B₂T-3D immunized pigs and high levels of IgG antibodies were maintained until day 70 (2 months pi) without significant differences between the two groups. As expected, no specific antibodies were detected in the sera from control PBS-inoculated pigs (Figure 1A).

Next, the ability of these antibodies to *in vitro* neutralize homologous virus was tested. B₂T-3A-immunized pigs elicited nAb by day 14 (1.3 ± 0.4 log10) that increased by day 21 (1.6 ± 0.6 log₁₀). After the second peptide dose, the titers increased reaching an average value of 2 ± 0.1 log₁₀ at day 28 pi, and a peak at day 35 pi (2.4 ± 0.1 logio), following a gradual smooth decrease until day 70 pi (1.8 ± 0.4 log₁₀) (Figure 1B).

The nAbs from B₂T-3D vaccinated group followed a similar time course and no significant differences were found when compared with B₂T-3A. At day 14 pi, nAbs titers were first observed (1.4 ± 0.1 log10) and increased at day 21 pi (1.8 ± 0.3 log10). After the boost, the average titers reached the peak at day 28 pi (2.1 ± 0.3 log10) and were maintained until day 35 pi (2.1 ± 0.3 log10). A slight decrease, similar to that observed in the B₂T-3A group, was detected at day 70 pi (1.5 ± 0.1 log10) (Figure 1C). No neutralizing activity was found in sera from PBS-inoculated animals at any time point (Figure 1D).

Thus, B₂T-3D elicited an antibody response in pigs that paralleled that of B₂T-3A.

### 2.1.2. T-cell responses elicited by B₂T-3D and B₂T-3A

The capacity of peptide B₂T-3D to induce specific T-cell responses was assessed in PBMCs isolated from immunized pigs by ELISPOT analysis of the IFN-γ-secreting cells. In this experiment, the B-cell peptide was included as stimulus for the *in vitro* recall, to address the possibility of its recognition by T-cells. As in previous experiments, intragroup variability was observed in the responses, which was reflected in the presence in each group of high responders (B₂T-3A: pigs 81 and 82; B₂T-3D: pigs 86 and 87) and low responders (B₂T-3A: pig 80; B₂T-3D: pigs 84 and 85). A remarkable primary response of IFN-γ secreting cells was noticed at day 14 pi (Figure 2). Interestingly, the two high responder pigs in the B₂T-3D group showed more IFN-γ spots than the higher responders in the B₂T-3A group when their PBMCs were stimulated with the whole homologous dendrimer (1743 ± 364 for B₂T-3D group vs 1206 ± 244 for B₂T-3A group) and the specific T-cell epitope (1679 ± 453 vs 959 ± 587). The magnitude of the responses was lower when cells were stimulated with the B-cell peptide, being the values higher in pigs immunized with B₂T-3A (130 ± 97 vs 507 ± 183) (Figure 2). At day 21 pi the response weaned in both groups reaching similar levels of IFN-γ spots when cells were stimulated with the dendrimer (847 ± 105 *vs* 800 ± 224) and the T-cell epitope (785 ± 5 vs 646 ± 382). At this time, responses against B-cell peptide were clearly lower (42 ± 16 vs 286 ± 186) (Figure 2). After the boost, a non-immediate secondary response was observed at day 35 pi in the two major responders in each group when stimulated with the corresponding dendrimer (1073 ± 132 vs 1161 ± 87, respectively). However, when stimulated with the T-cell peptide, the IFN-γ production was higher in the B₂T-3D group (929 ± 242 vs 637 ± 52). The response dramatically weaned in both groups being scarcely detected at day 70 pi. As expected, non-immunized animals did not induce IFN-γ secreting cells upon stimulation with any of the specific peptides (Figure 2).

Interestingly, the frequencies of IFN-γ spots in response to the B-cell peptide in pigs from the B₂T-3D group were considerably lower than those of B₂T-3A immunized animals. These results suggest that the B-cell epitope plays a minor role in cytokine production in B₂T-3D immunized pigs becoming T-3D epitope a more potent inducer of IFN-γ-producing cells compared to T-3A epitope.

### 2.1.3. Dendrimers B₂T-3D and B₂T-3A elicit nAbs against a broad spectrum of type O FMDVs

To assess the neutralization range afforded by B2T-3D and B2T-3A dendrimers, sera recovered from the pigs vaccinated in this study with peptides B₂T-3A and B₂T-3D, as well as those of pigs previously immunized with B₂T-3A (Canas-Arranz et al., 2020, A Single Dose of Dendrimer B2T Peptide Vaccine Partially Protects Pigs against Foot-and-Mouth Disease Virus Infection. Vaccines (Basel) 8; Canas-Arranz et al., 2020, A bivalent B-cell epitope dendrimer peptide can confer long-lasting immunity in swine against foot-and-mouth disease. Transbound Emerg Dis.) were tested for its capability to neutralize a panel of type O FMDVs (Figure 3). The FMDV isolates selected belonged to different type O topotypes, i.e viruses from different spatiotemporal locations.

At day 21 pi, animals immunized with B₂T-3A and B₂T-3D showed nAbs titers against the panel of FMDVs without significant differences among the viruses compared (Figure 3A). A similar neutralizing profile was observed at day 28 pi, after the second immunization with the same dose of each of the peptides (Figure 3B). The level of neutralization afforded at 21 dpi by B₂T-3A and B₂T-3D against the panel of viruses relative to the homologous isolate O/UK/11/2001 (r1 value; VNT ratio: virus problem/ O/UK/11/2001) is shown in Figure 3 (C and D). While for B₂T-3D the data compared belong to the four pigs immunized in this study (Figure 3D), for B₂T-3A, a total of 18 animals, including those immunized with this dendrimer in previous works (Canas-Arranz et al., 2020, A Single Dose of Dendrimer B2T Peptide Vaccine Partially Protects Pigs against Foot-and-Mouth Disease Virus Infection. Vaccines (Basel) 8; Canas-Arranz et al., 2020, A bivalent B-cell epitope dendrimer peptide can confer long-lasting immunity in swine against foot-and-mouth disease. Transbound Emerg Dis.) were included (Fig. 3C). All the viruses tested showed r1 values similar or for some of the animals immunized with B₂T-3A even higher than that of O/UK/11/2001.

These results support that B₂T-dendrimers induce a broad anti-FMDV immunity within a serotype, which can be considered as an important valuable asset for their potential use in endemic countries where a wide spectrum of antigenic variable pools of FMDVs can circulate.

### 3. Discussion

Both B₂T-3A and B₂T-3D elicited consistent levels of neutralizing antibodies. As previously reported, B₂T-3A also induced IFN-γ expressing T-cells that were *in vitro* recalled by T-3A peptide and, interestingly to a lower extent, by B-cell peptide with similar time courses, supporting that both sequences were recognized as T-cell epitopes. Conversely, the IFN-γ expressing cells elicited by B₂T-3D preferentially recognized the T-3D peptide, suggesting that this epitope is a potent inducer of IFN-γ. Our results show that dendrimer peptide B₂T-3D elicited, in most cases, high titers of cross-neutralizing antibodies, which, for some isolates, were higher than those against the homologous virus in a similar manner than when using B₂T-3A to immunized pigs. In summary, a B₂T-dendrimer incorporating FMDV T-cell epitope T-3D elicits high levels of neutralizing antibodies and a potent response of IFN-γ producing-cells.

## Claims

1. A dendrimeric peptide construct of formula (I) 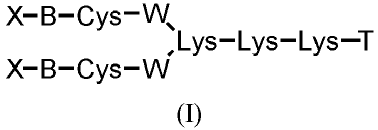 wherein
T is a T epitope of the non-structural protein 3D of the FMDV, bound to the lysine core by its N-terminal end;
B is a B epitope of the FMDV;
W is a linker group; and
X is an acyl group when B is bound to the α-amino group of the Cys by its C-terminal end, or is a -NH₂ group or is -OH when B is bound to the α-carboxylic group of the Cys by its N-terminal end.

2. The dendrimeric construct according to claim 1, wherein T is a T epitope of the FMDV comprising residues 56-70 of protein 3D of FMDV serotype C, or the corresponding amino acid residues of another serotype of FMDV.

3. The dendrimeric construct according to any one of the preceding claims, wherein T comprises the amino acid sequence:
IFSKH-AA₁-G-AA₂-AA₃-KM-AA₄-AA₅-AA₆-D
(SEQ ID NO: 1)
wherein AA₁ is an amino acid selected from R and K, AA₂ is amino acid selected from D and N, AA₃ is an amino acid selected from T and A, AA₄ is an amino acid selected from S and T, AA₅ is an amino acid selected from A, E, P and K, and AA₆ is an amino acid selected from E and A.

4. The dendrimeric construct according to claim 3, wherein T comprises the amino acid sequence:
IFSKH-AA₁-GDTKM-AA₄-A-AA₆-D
(SEQ ID NO: 2)
wherein AA₁ is an amino acid selected from R and K, AA₄ is an amino acid selected from S and T, and AA₆ is an amino acid selected from E and A.

5. The dendrimeric construct according to claim 4, wherein T comprises the amino acid sequence IFSKHRGDTKMSAED (SEQ ID NO: 3).

6. The dendrimeric construct according to any one of the preceding claims, wherein W is selected from the group consisting of: 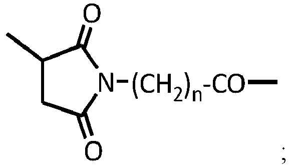 -(CH₂)ₙ-CO-; and
-CO-(CH₂)ₙ-S-(CH₂)ₘ-CO-;
wherein n and m are independently selected from 1, 2, 3, 4, 5 and 6.

7. The dendrimeric construct according to claim 6, wherein W is 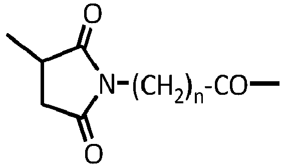 wherein n is selected from 1, 2, and 3.

8. The dendrimeric construct according to any one of the preceding claims, wherein B is a B epitope of the protein VP1 of the FMDV.

9. The dendrimeric construct according to claim 8, wherein B is a B epitope of the FMDV selected from the group consisting of residues 140-157 of protein VP1 of FMDV serotype O.

10. The dendrimeric construct according to any one of the preceding claims, wherein B epitope comprises of the amino acid sequence:
AA₇-AA₈-AA₉-AA₁₀-AA₁₁-RGDLQVLAQKAART
(SEQ ID NO: 4)
wherein AA₇ is an amino acid selected from P, S and A, AA₈ is an amino acid selected from V, M, A, T and E, AA₉ is an amino acid selected from T and A, AA₁₀ is an amino acid selected from N and S, and AA₁₁ is an amino acid selected from V and L.

11. The dendrimeric construct according to claim 10, wherein B comprises the amino acid sequence PVTNVRGDLQVLAQKAART (SEQ ID NO: 5).

12. The dendrimeric construct according to any one of the preceding claims which is a construct of formula (Ia): 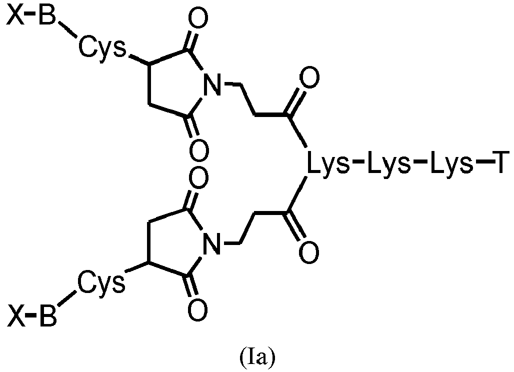
wherein X is an acyl group when B is bound to the α-amino group of the Cys by its C-terminal end, or is a -NH₂ group when B is bound to the α-carboxylic group of the Cys by its N-terminal end, and
B and T are as defined in any of claim 1-5 and 8-10.

13. The dendrimeric construct according to claim 12, wherein X is an acyl group, B is bound to the α-amino group of the Cys by its C-terminal end, B consists of the amino acid sequence PVTNVRGDLQVLAQKAART (SEQ ID NO: 5), and T consists of the amino acid sequence IFSKHRGDTKMSAED (SEQ ID NO: 3) capped at the C-terminus as a carboxamide.

14. A veterinary composition comprising a dendrimeric peptide construct of formula (I) as defined in any of claims 1 to 12 and a veterinary acceptable excipient.

15. A dendrimeric peptide construct of formula (I) as defined in any of claims 1 to 12 for use in medicine.

16. A dendrimeric peptide construct of formula (I) as defined in any of claims 1 to 12 for use in the prevention of foot-and-mouth disease in an animal.


SEQUENCE LISTING

<110>  CONSEJO SUPERIOR DE INVESTIGACIONES CIENTÍFICAS
       INSTITUTO NACIONAL DE INVESTIGACIÓN Y TECNOLOGÍA AGRARIA Y
       ALIMENTARIA (INIA)
       UNIVERSITAT POMPEU FABRA (UPF)

<120>  PEPTIDE VACCINES FOR THE PREVENTION OF FOOT-AND-MOUTH DISEASE

<130>  P19481EP00

<160>  19

<170>  PatentIn version 3.5

<210>  1
<211>  15
<212>  PRT
<213>  Foot-and-mouth disease virus



<220>
<221>  VARIANT
<222>  (6)..(6)
<223>  /replace= "Lys"

<220>
<221>  VARIANT
<222>  (8)..(8)
<223>  /replace= "Asn"

<220>
<221>  VARIANT
<222>  (9)..(9)
<223>  /replace= "Ala"

<220>
<221>  VARIANT
<222>  (12)..(12)
<223>  /replace= "Thr"

<220>
<221>  VARIANT
<222>  (13)..(13)
<223>  /replace= "Glu" /replace= "Pro" /replace= "Lys"

<220>
<221>  VARIANT
<222>  (14)..(14)
<223>  /replace= "Ala"

<400>  1

Ile Phe Ser Lys His Arg Gly Asp Thr Lys Met Ser Ala Glu Asp
1               5                   10                  15


<210>  2
<211>  15
<212>  PRT
<213>  Foot-and-mouth disease virus

<220>
<221>  VARIANT
<222>  (6)..(6)
<223>  /replace= "Lys"

<220>
<221>  VARIANT
<222>  (12)..(12)
<223>  /replace= "Thr"

<220>
<221>  VARIANT
<222>  (14)..(14)
<223>  /replace= "Ala"

<400>  2

Ile Phe Ser Lys His Arg Gly Asp Thr Lys Met Ser Ala Glu Asp
1               5                   10                  15


<210>  3
<211>  15
<212>  PRT
<213>  Foot-and-mouth disease virus

<400>  3

Ile Phe Ser Lys His Arg Gly Asp Thr Lys Met Ser Ala Glu Asp
1               5                   10                  15


<210>  4
<211>  19
<212>  PRT
<213>  Foot-and-mouth disease virus


<220>
<221>  VARIANT
<222>  (1)..(1)
<223>  /replace= "Ser" /replace= "Ala"

<220>
<221>  VARIANT
<222>  (2)..(2)
<223>  /replace= "Met" /replace= "Ala" /replace= "Thr" /replace= "Glu"

<220>
<221>  VARIANT
<222>  (3)..(3)
<223>  /replace= "Ala"

<220>
<221>  VARIANT
<222>  (4)..(4)
<223>  /replace= "Ser"

<220>
<221>  VARIANT
<222>  (5)..(5)
<223>  /replace= "Leu"

<400>  4

Pro Val Thr Asn Val Arg Gly Asp Leu Gln Val Leu Ala Gln Lys Ala
1               5                   10                  15

Ala Arg Thr

<210>  5
<211>  19
<212>  PRT
<213>  Foot-and-mouth disease virus

<400>  5

Pro Val Thr Asn Val Arg Gly Asp Leu Gln Val Leu Ala Gln Lys Ala
1               5                   10                  15

Ala Arg Thr

<210>  6
<211>  15
<212>  PRT
<213>  Foot-and-mouth disease virus

<400>  6

Ala Ala Ile Glu Phe Phe Glu Gly Met Val His Asp Ser Ile Lys
1               5                   10                  15

<210>  7
<211>  15
<212>  PRT
<213>  Foot-and-mouth disease virus

<400>  7

Ile Phe Ser Lys His Lys Gly Asp Thr Lys Met Ser Ala Glu Asp
1               5                   10                  15

<210>  8
<211>  15
<212>  PRT
<213>  Foot-and-mouth disease virus

<400>  8

Ile Phe Ser Lys His Lys Gly Asn Thr Lys Met Ser Glu Glu Asp
1               5                   10                  15

<210>  9
<211>  15
<212>  PRT
<213>  Foot-and-mouth disease virus

<400>  9

Ile Phe Ser Lys His Lys Gly Asp Thr Lys Met Ser Glu Glu Asp
1               5                   10                  15


<210>  10
<211>  15
<212>  PRT
<213>  Foot-and-mouth disease virus

<400>  10

Ile Phe Ser Lys His Lys Gly Asp Thr Lys Met Thr Pro Glu Asp
1               5                   10                  15


<210>  11
<211>  15
<212>  PRT
<213>  Foot-and-mouth disease virus

<400>  11

Ile Phe Ser Lys His Lys Gly Asp Thr Lys Met Ser Lys Glu Asp
1               5                   10                  15


<210>  12
<211>  15
<212>  PRT
<213>  Foot-and-mouth disease virus

<400>  12

Ile Phe Ser Lys His Lys Gly Asp Ala Lys Met Ser Glu Ala Asp
1               5                   10                  15


<210>  13
<211>  15
<212>  PRT
<213>  Foot-and-mouth disease virus

<400>  13

Ile Phe Ser Lys His Lys Gly Asp Thr Lys Met Thr Glu Glu Asp
1               5                   10                  15


<210>  14
<211>  19
<212>  PRT
<213>  Foot-and-mouth disease virus

<400>  14

Pro Val Thr Asn Val Arg Gly Asp Leu Gln Val Leu Ala Gln Lys Ala
1               5                   10                  15


Ala Arg Thr

<210>  15
<211>  19
<212>  PRT
<213>  Foot-and-mouth disease virus

<400>  15

Thr Val Ala Asn Val Arg Gly Asp Leu Gln Val Leu Ala Gln Lys Ala
1               5                   10                  15

Ala Arg Ala

<210>  16
<211>  19
<212>  PRT
<213>  Foot-and-mouth disease virus

<400>  16

Pro Thr Thr Asn Val Arg Gly Asp Leu Gln Val Leu Ala Gln Lys Ala
1               5                   10                  15

Ala Arg Thr

<210>  17
<211>  19
<212>  PRT
<213>  Foot-and-mouth disease virus

<400>  17

Pro Thr Thr Asn Val Arg Gly Asp Leu Gln Val Leu Ala Gln Lys Ala
1               5                   10                  15

Ala Arg Met

<210>  18
<211>  19
<212>  PRT
<213>  Foot-and-mouth disease virus

<400>  18

Ala Val Pro Asn Val Arg Gly Asp Leu Gln Val Leu Ala Gln Lys Val
1               5                   10                  15

Val Arg Thr

<210>  19

<211>  19
<212>  PRT
<213>  Foot-and-mouth disease virus

<400>  19

Ala Val Pro Asn Leu Arg Gly Asp Leu Gln Val Leu Ala Gln Lys Val
1               5                   10                  15

Ala Arg Thr